# EUROPEAN PATENT APPLICATION

(11) **EP 4 299 572 A1**
(43) Date of publication of application: **03.01.2024**
(21) Application number: 22760110.1
(22) Date of filing: 25.02.2022
(51) Int. Cl.: C07D 403/06, A61K 31/5377, A61K 31/4025, A61P 3/04, A61P 3/10, A61P 29/00, A61P 15/10

(54) **MELANOCORTIN-4 RECEPTOR AGONIST**

(30) Priority: 26.02.2021 KR 20210026468
(71) Applicant: Lg Chem, Ltd., Seoul 07336 (KR)
(72) Inventor: CHOI, Eun Sil, Seoul 07796 (KR); PARK, Hee Dong, Seoul 07796 (KR); KANG, Seung Wan, Seoul 07796 (KR); AHN, Hye Won, Seoul 07796 (KR); PARK, Deok Seong, Seoul 07796 (KR)
(74) Representative: Hoffmann Eitle
(86) International application number: PCT/KR2022/002782
(87) International publication number: WO 2022/182194

(57) **Abstract**

The present invention relates to a compound showing an excellent agonistic activity for a melanocortin receptor and, more specifically, to the compound of chemical formula 1, a pharmaceutical composition containing same as an active ingredient, and a use thereof. In detail, the compound of the present invention exhibits an excellent agonistic activity for melanocortin-4 receptor and thus, can be advantageously used especially for preventing or treating obesity, diabetes, inflammation, and impotence.

## Description

### TECHNICAL FIELD

The present invention relates to a compound exhibiting excellent agonist activity against melanocortin receptors. More specifically, the present invention relates to a compound of the following Formula 1, a pharmaceutical composition comprising the compound as an active ingredient, and a use thereof, and the compound of the present invention exhibits excellent agonist activity against melanocortin-4 receptors and can be particularly useful in preventing or treating obesity, diabetes, inflammation and erectile dysfunction: 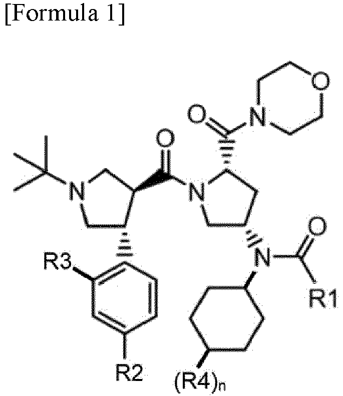 wherein R1, R2, R3, R4 and n are the same as defined herein.

### BACKGROUND ART

Leptin protein is a hormone secreted by body fat cells (adipocytes), and the amount of secretion thereof increases as the body fat content increases. The leptin protein regulates the functions of various neuropeptides produced in the hypothalamus, thereby regulating appetite, the body fat content, and various *in vivo* functions including energy metabolism (Schwartz, et al., Nature 404, 661-671 (2000)). The signal transduction of appetite and weight control by leptin protein is made through the regulation of many factors downstream, the most representative of which are melanocortin, agouti-related peptide (AgRP), and neuropeptide Y (NPY) hormone.

When the concentration of leptin in the blood increases as a result of excessive calories *in vivo,* the secretion of proopiomelanocortin (POMC) protein hormone in the pituitary gland increases, and the production of AgRP and NPY decreases. Alpha-MSH (melanocyte stimulating hormone), which is a small peptide hormone, is produced from POMC neurons, and this hormone is an agonist of the melanocortin-4 receptor (MC4R) of secondary neurons and ultimately induces appetite reduction. On the other hand, when the concentration of leptin decreases due to calorie deficiency, the expression of AgRP-which is the MC4R antagonist-increases and the expression of NPY is also increased, this ultimately enhances appetite. That is, depending on the changes in leptin, the alpha-MSH hormone and the AgRP hormone are involved in appetite regulation by being an agonist and antagonist for MC4R.

Alpha-MSH hormone induces various physiological responses by binding to 3 MCR subtypes in addition to MC4R. Until now, five MCR subtypes have been identified. Among the subtypes, it is known that MC1R is mainly expressed in skin cells and is involved in melanin pigmentation, MC2R is mainly expressed in the adrenal gland and is involved in the production of glucocorticoid hormone, and only ACTH (adrenocorticotropic hormone) derived from POMC is the ligand thereof. MC3R and MC4R-which are mainly expressed in the central nervous system-are involved in the regulation of appetite, energy metabolism, and fat storage efficiency in the body, and MC5R-which is expressed in various tissues-is known to regulate exocrine function (Wikberg, et al., Pharm Res 42 (5) 393-420 (2000)). Specifically, the activation of the MC4R receptor has an effect of effectively reducing body weight by inducing a decrease in appetite and an increase in energy metabolism and thus is proven to be a major point of action in the development of obesity drugs (Review: Wikberg, Eur. J. Pharmacol 375, 295-310 (1999)); Wikberg, et al., Pharm Res 42 (5) 393-420 (2000); Douglas et al., Eur J Pharm 450, 93-109 (2002); and O'Rahilly et al., Nature Med 10, 351-352 (2004)).

The role of MC4R in appetite and weight control has been primarily demonstrated through an experiment on an animal model of agouti protein abnormal expression (agouti mouse). In the case of agouti mice, it is found that the agouti protein is expressed at high concentrations in the central nervous system and acts as an antagonist of MC4R in the hypothalamus due to genetic mutations, thus inducing obesity (Yen, TT et al., FASEB J. 8, 479-488 (1994); and Lu D., et al. Nature 371, 799-802 (1994)). In subsequent research results, it is observed that AgRP (agouti-related peptide) similar to the actual agouti protein was expressed in the hypothalamic nerve, and AgRP is also known to be involved in appetite regulation as antagonists against MC4R (Shutter, et al., Genes Dev., 11, 593-602 (1997); and Ollman, et al. Science 278, 135-138 (1997)).

Cerebral administration of alpha-MSH-which is an MC4R agonist *in vivo*-to an animal exhibits an effect of reducing appetite, and if SHU9119 (peptide) or HS014 (peptide)-which are MC4R antagonists-are treated thereto, an effect of increasing the appetite again is observed (Kask et al., Biochem. Biophys. Res. Comm. 245, 90-93 (1998)). In addition, in an animal test using Melanotan II (MTII, Ac-Nle-c[Asp-His-DPhe-Arg-Trp-Lys]-NH2) and HP228, which is an agonist similar thereto, after cerebral, intraperitoneal or subcutaneous administration, appetite suppression, weight loss, and energy metabolism increase efficacy, and the like were confirmed. (Thiele T. E., et al. Am J Physiol 274 (1 Pt 2), R248-54 (1998); Lee M. D., et al. FASEB J 12, A552 (1998); Murphy B., et al. J Appl Physiol 89, 273-82 (2000)). In contrast, when the representative SHU9119 is administered to an animal, significant and sustained feed intake and weight gain are exhibited, providing the pharmacological evidence that MCR agonists can be used to treat obesity. The appetite-reducing effect-which is evidently exhibited during the administration of MTII-is not exhibited in MC4R KO (knock-out) mice, and this experimental result again proves that the appetite-reducing effect is mainly achieved by the activation of MC4R (Marsh, et al., Nat Genet 21, 119-122 (1999)).

Appetite inhibitors that act on the central nervous system are predominant as obesity treatments developed up to date, and most of the inhibitors are drugs that modulate the action of neurotransmitters. Examples thereof include noradrenalin agents (phentermine and mazindol) and fluoxetine and sibutramine, which are serotonergic agents. However, the neurotransmitter modulator exerts a wide range of effects on various physiological actions in addition to appetite suppression by numerous subtype receptors. Therefore, the modulators lack in selectivity for each subtype and have a large disadvantage in that various side effects are accompanied in case of long-term administration.

On the other hand, melanocortins are neuropeptides, not neurotransmitters, and considering that all other functions other than energy metabolism are normal in the MC4R gene KO mice, melanocortin agonists have an advantage as an action point in that only weight loss through appetite inhibition can be induced without affecting other physiological functions. In particular, the receptor is a G-protein coupled receptor (GPCR), which belongs to the most successful category among the new drug action points developed so far, and is largely distinguished from the action points in the related art in that it is relatively easy to secure selectivity for subtype receptors.

As examples of utilizing the melanocortin receptor as an action point, International Publication Nos. WO 2008/007930 and WO 2010/056022 disclose compounds as agonists of the melanocortin receptor.

### DISCLOSURE OF INVENTION

### TECHNICAL PROBLEM

An object of the present invention is to provide a novel compound represented by Formula 1, which has excellent selective agonist activity against melanocortin receptors, specifically melanocortin-4 receptors (MC4R), or a pharmaceutically acceptable salt or isomer thereof.

Another object of the present invention is to provide a method of preparing the compound represented by Formula 1.

Still another object of the present invention is to provide a melanocortin receptor agonistic pharmaceutical composition comprising a compound represented by Formula 1, or a pharmaceutically acceptable salt or isomer thereof, as an active ingredient.

Still another object of the present invention is to provide use of the compound represented by Formula 1, or a pharmaceutically acceptable salt or isomer thereof in the prevention or treatment of obesity, diabetes, inflammation and erectile dysfunction.

### SOLUTION TO PROBLEM

In order to achieve the above object, the present invention provides a compound of the following Formula 1, or a pharmaceutically acceptable salt or isomer thereof: 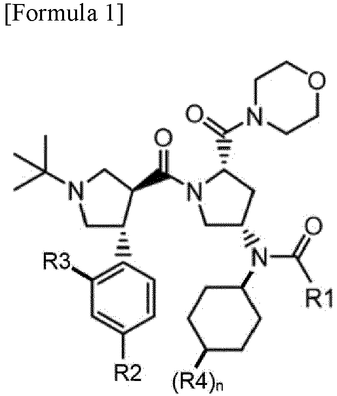 wherein
R1 is C₂-C₅ alkyl;
R2 is halo;
R3 is hydrogen or halo;
R4 is C₁-C₃ alkyl; and
n is an integer of 1 or 2;
provided that n is 2, when R2 is chloride and R3 is hydrogen.

The compound of Formula 1 according to the present invention may form a pharmaceutically acceptable salt.

In addition, since the compounds according to the present invention may have an asymmetric carbon center and an asymmetric axis or an asymmetric plane, the compounds may exist as cis or trans isomers, R or S isomers, racemates, diastereomer mixtures, and individual diastereomers, and all these isomers and mixtures are included within the scope of the present invention.

In the present specification, unless indicated otherwise, the compound of Formula 1 is used in the sense of including all of the compound of Formula 1, pharmaceutically acceptable salts and isomers thereof.

The term "halo" used herein means a radical of fluoride (F), chlorine (Cl), bromine (Br) or iodine (I).

The term "alkyl" used herein means a linear or branched hydrocarbon group.

In one embodiment according to the present invention, R1 in Formula 1 is C₂ to C₄ alkyl. In another embodiment according to the present invention, R1 in Formula 1 is a linear or branched C₂ to C₄ alkyl-for example, ethyl, n-propyl, isopropyl, n-butyl, isobutyl, sec-butyl, or tert-butyl.

In another embodiment according to the present invention, R1 in Formula 1 is C₃ or C₄ alkyl. In another embodiment according to the present invention, R1 in Formula 1 is branched C₃ or C₄ alkyl-for example, isopropyl or tert-butyl.

In another embodiment according to the present invention, the compound of Formula 1 is *N*-((3*S*,5*S*)-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N*-((1*s*,4*R*)-4-methylcyclohexyl)pivalamide of the following Formula 2:

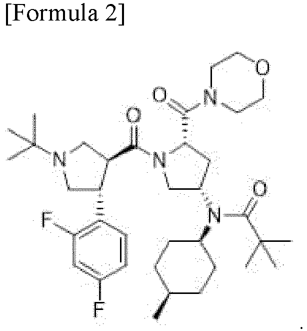

In another embodiment according to the present invention, the compound of Formula 1 is *N*-((3*S*,5*S*)-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N*-((1*s*,4*R*)-4-methylcyclohexyl)isobutyramide of the following Formula 3:

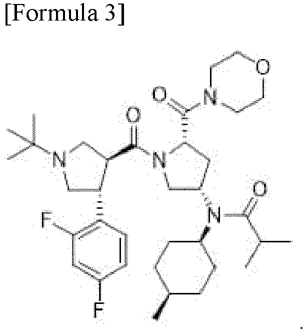

In another embodiment according to the present invention, the compound of Formula 1 is *N-*((3*S*,5*S*)-1-((3*S*,4*R*)-1-(*tert*-butyl)-4-(4-chloro-2-fluorophenyl)pyrrolidine-3-carbonyl)-5-(morpholine-4-carbonyl)pyrrolidin-3-yl)-*N-*((1s,4R)-4-methylcyclohexyl)isobutyramide of the following Formula 4:

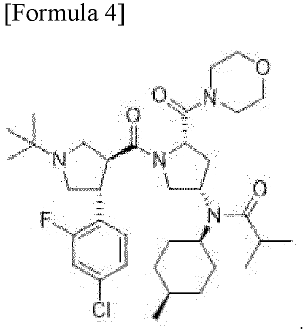

In another embodiment according to the present invention, the compound of Formula 1 is *N-*((3*S*,5*S*)-1-((3*S*,4*R*)-1-(*tert*-butyl)-4-(4-chloro-2-fluorophenyl)pyrrolidine-3-carbonyl)-5-(morpholine-4-carbonyl)pyrrolidin-3-yl)-*N-*((1s,4R)-4-methylcyclohexyl)pivalamide of the following Formula 5:

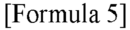

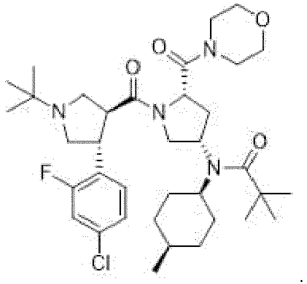

In another embodiment according to the present invention, the compound of Formula 1 is *N*-((3*S*,5*S*)-1-((3*S*,4*R*)-1-(*tert-*butyl)-4-(4-chlorophenyl)pyrrolidine-3-carbonyl)-5-(morpholine-4-carbonyl)pyrrolidin-3-yl)-*N*-(4,4-dimethylcyclohexyl)isobutyramide of the following Formula 6:

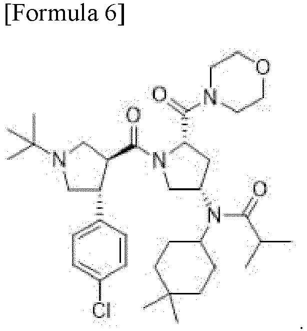

In another embodiment according to the present invention, the compound of Formula 1 is *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-(4,4-dimethylcyclohexyl)pivalamide of the following Formula 7:

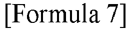

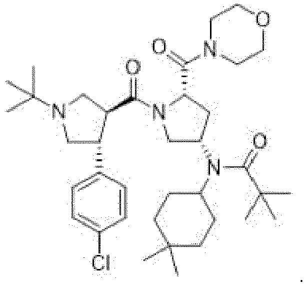

In another embodiment according to the present invention, examples of the pharmaceutically acceptable salt include acid addition salts formed by an inorganic acid such as hydrochloric acid, sulfuric acid, nitric acid, phosphoric acid, hydrobromic acid, and hydroiodic acid; an organic carboxylic acid such as tartaric acid, formic acid, citric acid, acetic acid, trichloroacetic acid, trifluoroacetic acid, gluconic acid, benzoic acid, lactic acid, fumaric acid, and maleic acid; and sulfonic acid such as methanesulfonic acid, benzenesulfonic acid, p-toluenesulfonic acid, and naphthalenesulfonic acid, but is not limited thereto.

In another embodiment according to the present invention, the pharmaceutically acceptable salt is hydrochloride.

In another embodiment according to the present invention, the compound of Formula 1 is *N*-((3*S*,5*S*)-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N*-((1*s*,4*R*)-4-methylcyclohexyl)pivalamide hydrochloride of the following Formula 8:

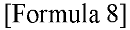

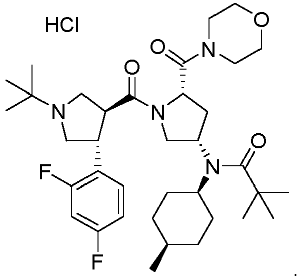

In another embodiment according to the present invention, the compound of Formula 1 is *N*-((3*S*,5*S*)-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N*-((1*s*,4*R*)-4-methylcyclohexyl)isobutyramide hydrochloride of the following Formula 9:

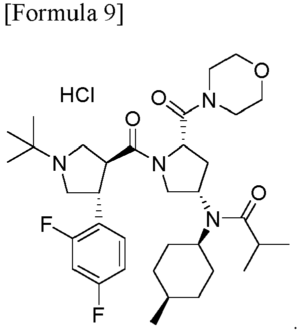

In another embodiment according to the present invention, the compound of Formula 1 is *N-*((3*S*,5*S*-1-((3*S*,4*R*)-1-(tert-butyl)-4-(4-chloro-2-fluorophenyl)pyrrolidine-3-carbonyl)-5-(morpholine-4-carbonyl)pyrrolidin-3-yl)-*N-*((1*s*,4*R*)-4-methylcyclohexyl)isobutyramide hydrochloride of the following Formula 10:

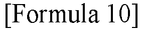

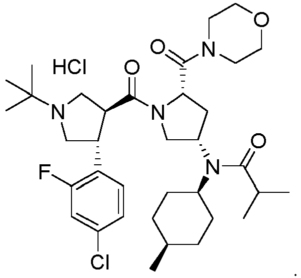

In another embodiment according to the present invention, the compound of Formula 1 is *N-*((3*S*,5*S*)-1-((3S,4R)-1-(*tert*-butyl)-4-(4-chloro-2-fluorophenyl)pyrrolidine-3-carbonyl)-5-(morpholine-4-carbonyl)pyrrolidin-3-yl)-*N-*((1*s*,4*R*)-4-methylcyclohexyl)pivalamide of the following Formula 11:

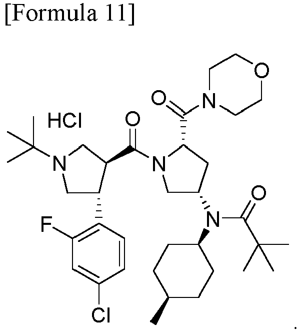

In another embodiment according to the present invention, the compound of Formula 1 is *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-(4,4-dimethylcyclohexyl)isobutyramide hydrochloride of the following Formula 12:

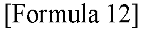

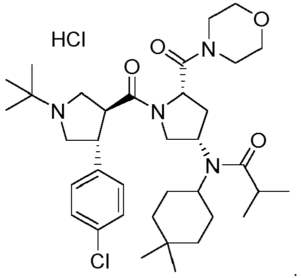

In another embodiment according to the present invention, the compound of Formula 1 is *N*-((3*S*,5*S*)-1-((3*S,*4*R*)-1-(*tert*-butyl)-4-(4-chlorophenyl)pyrrolidine-3-carbonyl)-5-(morpholine-4-carbonyl)pyrrolidin-3-yl)-*N*-(4,4-dimethylcyclohexyl)pivalamide hydrochloride of the following Formula 13:

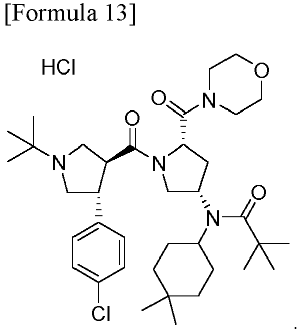

In another embodiment according to the present invention, *N*-((3*S*,5*S*)-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N*-((1*s*,4*R*)-4-methylcyclohexyl)pivalamide hydrochloride of the above Formula 8, *N*-((3S,5*S*-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N-*((1*s*,4*R*)-4-methylcyclohexyl)isobutyramide hydrochloride of the above Formula 9, *N-*((3*S*,5*S*)-1-((3*S*,4*R*)-1-(*tert*-butyl)-4-(4-chloro-2-fluorophenyl)pyrrolidine-3-carbonyl)-5-(morpholine-4-carbonyl)pyrrolidin-3-yl)-*N*-((1*s*,4*R*)-4-methylcyclohexyl)isobutyramide hydrochloride of the above Formula 10 and *N*-((3*S*,5*S*)-1-((3*S*,4*R*)-1-(*tert*-butyl)-4-(4-chloro-2-fluorophenyl)pyrrolidine-3-carbonyl)-5-(morpholine-4-carbonyl)pyrrolidin-3-yl)-*N-*((1*s*,4*R*)-4-methylcyclohexyl)pivalamide of the above Formula 11 may be prepared according to the following Reaction Scheme 1. 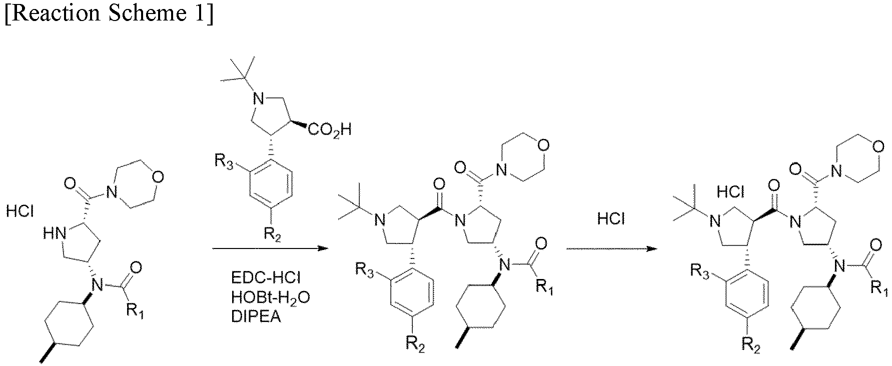 in Reaction Scheme 1,
R1, R2 and R3 are the same as defined in the above.

In another embodiment according to the present invention, *N-*((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-(4,4-dimethylcyclohexyl)isobutyramide hydrochloride of the above Formula 12 and *N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N-*(4,4-dimethylcyclohexyl)pivalamide hydrochloride of the above Formula 13 may be prepared according to the following Reaction Scheme 2. 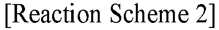 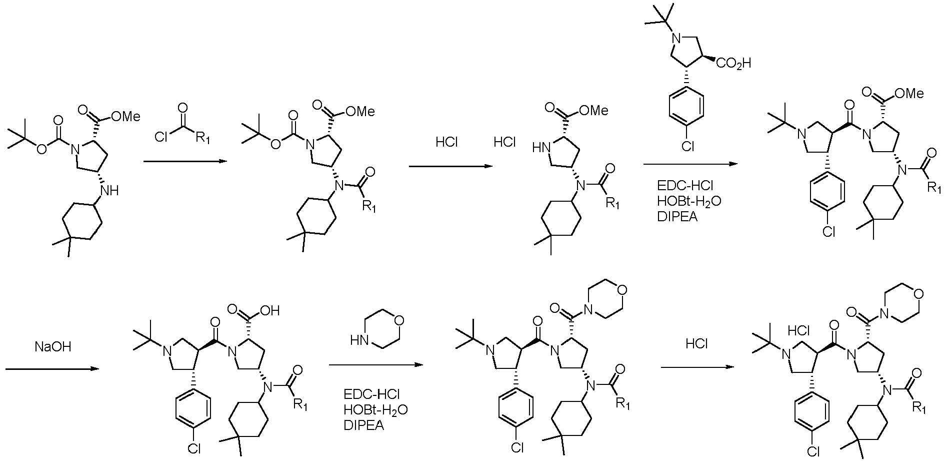 in Reaction Scheme 2,
R1 is the same as defined in the above.

The compound of Formula 1 according to the present invention exhibits excellent agonist activity against melanocortin receptors, specifically melanocortin-4 receptors (MC4R), and thus the present invention also provides a melanocortin receptor agonistic pharmaceutical composition comprising the compound of Formula 1, or a pharmaceutically acceptable salt or isomer thereof as an active ingredient, together with a pharmaceutically acceptable carrier. In particular, the composition according to the present invention exhibits an excellent effect in preventing or treating obesity, diabetes, inflammation and erectile dysfunction, but is not limited thereto.

In the present specification, the "carrier" refers to a compound that facilitates the injection of the compound into cells or tissues.

When the compound of the present invention is administered for clinical purposes, the total daily dose to be administered to a host as a single dose or in separate doses is preferably in the range of 0.01 to 10 mg per 1 kg of the body weight, but a specific dose level for individual patients may vary depending on the specific compound to be used, the weight, the sex, the health status, and the diet of the patient, the administration time, the administration method, and the excretion rate of the drug, the drug mixture, and the severity of a disease.

The compound of the present invention can be administered by any route depending on the purpose. For example, the compound of the present invention can be administered by injection or oral administration.

Formulations for injection can be produced by using a suitable dispersing agent, wetting agent, or suspending agent according to known techniques.

Examples of solid dosage forms for oral administration include capsules, tablets, pills, powders, and granules, and solid dosage forms may be produced by mixing the active compound of Formula 1 according to the present invention with one or more carriers such as inert diluents, lubricants, disintegrants and binders.

### EFFECTS OF THE INVENTION

The compound of Formula 1 according to the present invention exhibits excellent agonist activity against melanocortin receptors, particularly melanocortin-4 receptors (MC4R), and thus can be usefully used in the prevention or treatment of obesity, diabetes, inflammation, and erectile dysfunction.

The compound of Formula 1 according to the present invention exhibits an on-target effect on the melanocortin-4 receptor, does not effect anxiety and depression while exhibiting weight loss and diet reduction effects, and can be administered without side effects on human ether-a-go-go related gene (hERG) inhibition or safety problems such as mutagenesis. In addition, the compound of Formula 1 according to the present invention can be safely administered because there is no cytotoxicity and liver toxicity.

### MODE FOR THE INVENTION

Hereinafter, the present invention is explained in more detail with the following examples. However, it must be understood that the protection scope of the present invention is not limited to the examples.

### Preparation Example 1: Preparation of N-((1s,4R)-4-methylcyclohexyl)-N-((3S,5S)-5-(morpholine-4-carbonyl)pyrrolidin-3-yl)pivalamide hydrochloride

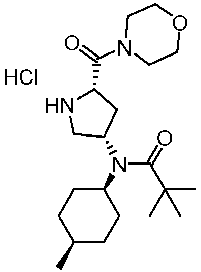

The title compound was obtained through the following Steps A, B and C.

### Step A: Preparation of (2S,4S)-1-(tert-butoxycarbonyl)-4-(N-((1s,4R-methylcyclohexyl)pivalamido)pyrrolidine-2-carboxylic acid

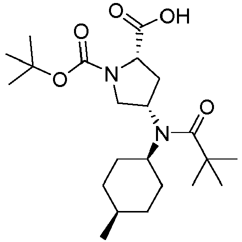

The title compound was obtained by the method disclosed in International Publication No. WO 2008/007930.
MS [M+Na] = 433.4 (M+23)
¹H NMR (400 MHz, CD₃OD) δ 4.25 (m, 1H), 3.86 (m, 2H), 3.42 (m, 2H), 2.80 (m, 1H), 2.27 (m, 1H), 2.00-1.80 (m, 3H), 1.66 (m, 4H), 1.43 (m, 11H), 1.26 (m, 9H), 1.05 (d, 3H)

### Step B: Preparation of tert-butyl (2S,4S)-4-(N-((1s,4R)-4-methylcyclohexyl)pivalamido)-2-(morpholine-4-carbonyl)pyrrolidine-1-carboxylate

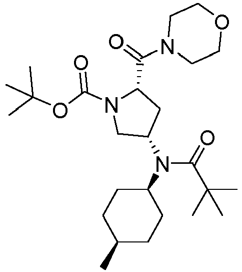

(2*S*,4*S*)-1-(*tert*-butoxycarbonyl)-4-(*N*-((1*s*,4*R*)-4-methylcyclohexyl)pivalamido)pyrrolidine-2-carboxylic acid (0.81 g, 1.97 mmol) obtained in Step A was dissolved in 10 mL of dimethylformamide, and morpholine (0.19 mL, 2.17 mmol), 1*H*-benzo[*d*][1,2,3]triazol-1-ol 1 hydrate (0.36 g, 2.36 mmol), 3-(((ethylimino)methylene)amino)-*N,N-*dimethylpropan-1-amine hydrochloride (0.45 g, 2.36 mmol) and *N,N*-diisopropylethylamine (1.0 mL, 5.92 mmol) were added thereto and stirred at room temperature for 16 hours. After the reaction was completed, the reaction solution was concentrated under reduced pressure, and the filtrate was washed with an aqueous sodium bicarbonate solution and extracted with ethyl acetate. The organic layers were collected, washed with water, dried over anhydrous magnesium sulfate, and the solid was filtered. The filtrate was concentrated under reduced pressure and purified by column chromatography to obtain the title compound (0.62 g, 66%).
MS [M+H] = 480.5 (M+1)
¹H NMR (400 MHz, CD₃OD) δ 4.65 (m, 1H), 4.00-3.40 (m, 12H), 2.82 (m, 1H), 2.22 (m, 1H), 2.00-1.80 (m, 3H), 1.70-1.60 (m, 4H), 1.50-1.41 (m, 11H), 1.24 (s, 9H), 1.04 (d, 3H)

### Step C: Preparation of N-((1s,4R)-4-methylcyclohexyl)-N-((3S,5S)-5-(morpholine-4-carbonyl)pyrrolidin-3-yl)pivalamide hydrochloride

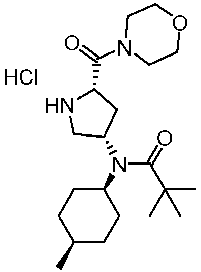

Tert-butyl (2*S*,4*S*)-4-(*N*-((1*s*,4*R*)-4-methylcyclohexyl)pivalamido)-2-(morpholine-4-carbonyl)pyrrolidine-1-carboxylate (0.62 g, 1.3 mmol) obtained in Step B was dissolved in 4 mL of dichloromethane, and 4 M hydrochloric acid in 1,4-dioxane solution (1.3 mL, 5.1 mmol) was added thereto and stirred at room temperature for 16 hours. After the reaction solution was concentrated under reduced pressure, the resulting solid was washed with diethyl ether and dried to obtain the title compound (0.55 g, 99%).
MS [M+H] = 380.6 (M+1)
¹H NMR (400 MHz, DMSO-d₆) δ 10.00-8.00 (brs, 2H), 4.46 (m, 1H), 4.14 (m, 1H), 3.76 (m, 1H), 3.65-3.25 (m, 10H), 2.47 (m, 1H), 1.99 (m, 1H), 1.91 (m, 1H), 1.80-1.50 (m, 6H), 1.39 (m, 2H), 1.19 (s, 9H), 1.00 (d, 3H)

### Preparation Example 2: Preparation of N-((1s,4R)-4-methylcyclohexyl)-N-((3S,5S)-5-(morpholine-4-carbonyl)pyrrolidin-3-yl)isobutyramide hydrochloride

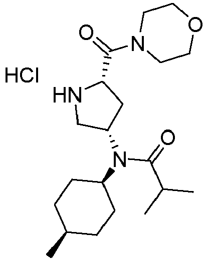

The title compound was obtained through the following Steps A, B, C, D, E, F and G.

### Step A: Preparation of 1-(tert-butyl) 2-methyl (2S,4S)-4-azidopyrrolidine-1,2-dicarboxylate

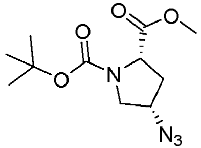

1-(*Tert*-butyl) 2-methyl (2*S*,4*R*)-4-((methyl sulfonyl)oxy)pyrrolidine-1,2-dicarboxylate (48.5 g, 150 mmol) was dissolved in 250 mL of dimethylformamide, and sodium azide (19.5 g, 300 mmol) was added thereto. After the mixture solution was stirred at 80°C for 16 hours, the solution was concentrated under reduced pressure. Water was added thereto, followed by extraction with ethyl acetate. The organic layer was washed with an aqueous sodium chloride solution and water, dried over anhydrous magnesium sulfate and filtered. The filtrate was concentrated under reduced pressure to obtain the title compound (39.59 g, 98%), which was used in the next step without purification.
MS [M+H] = 271 (M+1)
¹H NMR (400 MHz, CD₃OD) δ 4.43-4.37 (m, 1H), 4.35-4.27 (br, 1H), 3.77 (s, 1.8H), 3.76 (s, 1.2H), 3.73-3.66 (m, 1H), 3.44-3.38 (m, 1H), 2.63-2.49 (m, 1H), 2.19-2.11 (m, 1H), 1.50 (s, 4.5H), 1.44 (s, 4.5H)

### Step B: Preparation of 1-(tert-butyl) 2-methyl (2S,4S)-4-aminopyrrolidine-1,2-dicarboxylate

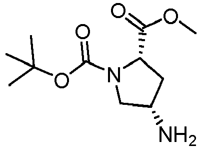

1-(*Tert*-butyl) 2-methyl (2*S*,4*S*)-4-azidopyrrolidine-1,2-dicarboxylate (24.6 g, 91.0 mmol) obtained in Step A was dissolved in 180 mL of tetrahydrofuran, and 1 M trimethylphosphine tetrahydrofuran solution (109 mL, 109 mmol) was slowly added thereto at 0°C. After stirring at the same temperature for 1 hour, the reaction mixture was stirred at room temperature for 3 hours. After the reaction solvent was concentrated under reduced pressure, 100 mL of dichloromethane and 150 mL of water were added, and the reaction mixture was stirred for about 30 minutes. The organic layer was dried over anhydrous magnesium sulfate, and the solid was filtered. The filtrate was concentrated under reduced pressure to obtain the title compound (20.62 g, 93%).
MS [M+H] = 245 (M+1)
¹H NMR (400 MHz, CD₃OD) δ 4.27 (m, 1H), 3.77 (s, 1.8H), 3.76 (s, 1.2H), 3.75-3.67 (m, 1H), 3.50-3.42 (m, 1H), 3.22-3.17 (m, 1H), 2.58-2.47 (m, 1H), 1.82-1.71 (m, 1H), 1.48 (s, 4.5H), 1.42 (s, 4.5H)

### Step C: Preparation of 1-(tert-butyl) 2-methyl (2S,4S)-4-(((1s,4R)-4-methylcyclohexyl)amino)pyrrolidine-1,2-dicarboxylate

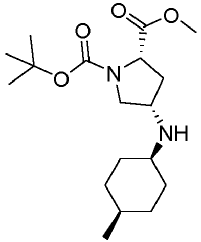

1-(*Tert*-butyl) 2-methyl (2*S*,4*S*)-4-aminopyrrolidine-1,2-dicarboxylate (20.6 g, 84.4 mmol) obtained in Step B was dissolved in 150 mL of 1,2-dichloroethane, and 4-methylcyclohexanone (9.50 mL, 101 mmol) was added thereto. After the mixture solution was cooled to 0°C, sodium triacetoxyborohydride (26.8 g, 127 mmol) was added, and the reaction mixture was stirred at room temperature for 16 hours. After the reaction solution was concentrated under reduced pressure, water was added and the reaction mixture was extracted with ethyl acetate. The organic layer was washed with an aqueous sodium chloride solution, dried over anhydrous magnesium sulfate and filtered. The filtrate was concentrated under reduced pressure and purified by column chromatography to obtain the title compound (22.9 g, 80%).
MS [M+H] = 341 (M+1)
¹H NMR (400 MHz, CD₃OD) δ 4.26 (m, 1H), 3.76 (s, 1.8H), 3.75 (s, 1.2H), 3.78-3.71 (m, 1H), 3.49-3.40 (m, 1H), 3.22-3.16 (m, 1H), 2.69-2.60 (br, 1H), 2.58-2.46 (m, 1H), 1.87-1.77 (m, 1H), 1.73-1.63 (m, 1H), 1.62-1.35 (m, 8H), 1.48 (s, 4.5H), 1.42 (s, 4.5H), 0.96 (d, 3H)

### Step D: Preparation of 1-(tert-butyl) 2-methyl (2S,4S)-4-(N-((1s,4R)-4-methylcyclohexyl)isobutyramido(pyrrolidine-1,2-dicarboxylate

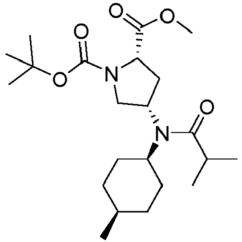

After dissolving 1-(*tert*-butyl) 2-methyl (2*S*,4*S*)-4-(((1*s*,4*R*)-4-methylcyclohexyl)amino)pyrrolidine-1,2-dicarboxylate (37.29 g, 109.5 mmol) obtained in Step C in 500 mL of dichloromethane, triethylamine (61.1 mL, 438 mmol) was added, and then isobutyryl chloride (11.7 mL, 219 mmol) was slowly added at 0°C. After stirring at room temperature for 16 hours, the reaction solvent was concentrated under reduced pressure. After adding sodium bicarbonate aqueous solution and ethyl acetate to the concentrate, the organic layer was separated. The organic layer was washed with an aqueous sodium chloride solution and water, dried over anhydrous magnesium sulfate and filtered. The filtrate was concentrated under reduced pressure and purified by column chromatography to obtain the title compound (38.79 g, 86%).
MS [M+H] = 411 (M+1)
¹H NMR (400 MHz, CD₃OD) δ 4.27 (m, 1H), 3.76 (s, 1.8H), 3.75 (s,1.2H), 3.78-3.72 (m, 1H), 3.50-3.41 (m, 1H), 3.33-3.14 (m, 1H), 2.69-2.60 (m, 2H), 2.57-2.43 (m, 1H), 1.87-1.79 (m, 1H), 1.70-1.61 (m, 1H), 1.60-1.32 (m, 8H), 1.47 (s, 4.5H), 1.41 (s, 4.5H), 1.10 (dd, 6H), 0.99 (d, 3H)

### Step E: Preparation of (2S,4S)-1-(tert-butoxycarbonyl)-4-(N-((1s,4R-methylcyclohexyl)isobutyramido)pyrrolidine-2-carboxylic acid

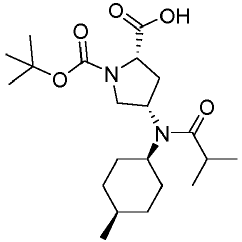

1-(*Tert*-butyl) 2-methyl (2*S*,4*S*)-4-(*N*-((1*s*,4*R*)-4-methylcyclohexyl)isobutyramido)pyrrolidine-1,2-dicarboxylate (3.63 g, 8.85 mmol) obtained in Step D was dissolved in 30 mL of ethanol, and 1 N aqueous sodium hydroxide solution (26.5 mL, 26.5 mmol) was added thereto and stirred at room temperature for 2 hours. After diluting the reaction solution with water, the pH was adjusted to 4 using a 1 N aqueous hydrochloric acid solution. The reaction mixture was extracted with ethyl acetate, and the organic layer was separated and dried over anhydrous sodium sulfate. The solid was filtered, and the filtrate was concentrated under reduced pressure to obtain the title compound (2.40 g, 69%).
MS [M+Na] = 419.4 (M+23)
¹H NMR (400 MHz, CD₃OD) δ 4.27 (m, 1H), 4.00-3.85 (m, 2H), 3.66 (m, 1H), 3.44 (m, 1H), 2.86 (m, 1H), 1.95-1.80 (m, 3H), 1.76-1.53 (m, 5H), 1.50-1.42 (m, 11H), 1.10-1.05 (m, 9H)

### Step F: Preparation of tert-butyl (2S,4S)-4-(N-((1s,4R)-4-methylcyclohexyl)isobutyramido)-2-(morpholine-4-carbonyl)pyrrolidine-1-carboxylate

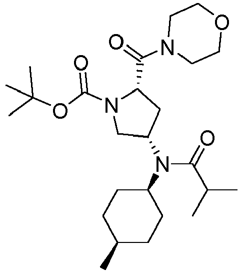

(2*S*,4*S*)-1-(*tert*-butoxycarbonyl)-4-(*N*-((1*s*,4*R*)-4-methylcyclohexyl)isobutyramido)pyrrolidine-2-carboxylic acid (2.40 g, 6.05 mmol) obtained in Step E, 1*H*-benzo[*d*][1,2,3]triazol-1-ol 1 hydrate (1.11 g, 7.26 mmol) and 3-(((ethylimino)methylene)amino)-*N*,*N*-dimethylpropan-1-amine hydrochloride (1.39 g, 7.26 mmol) were dissolved in 30 mL of dimethylformamide. Morpholine (0.55 mL, 6.66 mmol) and N,N-diisopropylethylamine (3.10 mL, 18.2 mmol) were added to the above mixture, followed by stirring at room temperature for 16 hours. After the reaction was completed, the reaction solution was concentrated under reduced pressure, and the filtrate was washed with an aqueous sodium bicarbonate solution and extracted with ethyl acetate. The organic layers were collected, washed with water and dried over anhydrous magnesium sulfate, and the solid was filtered. The filtrate was concentrated under reduced pressure and purified by column chromatography to obtain the title compound (1.87 g, 66%).
MS [M+H] = 466.5 (M+1)
¹H NMR (400 MHz, CD₃OD) δ 4.66 (m, 1H), 3.94 (m, 1H), 3.75-3.55 (m, 10H), 3.45 (m, 1H), 2.86 (m, 1H), 2.24 (m, 1H), 1.95-1.80 (m, 3H), 1.77-1.60 (m, 5H), 1.59-1.45 (m, 2H), 1.46-1.41 (m, 9H), 1.05 (m, 9H)

### Step G: Preparation of N-((1s,4R)-4-methylcyclohexyl)-N-((3S,5S)-5-(morpholine-4-carbonyl)pyrrolidin-3-yl)isobutyramide hydrochloride

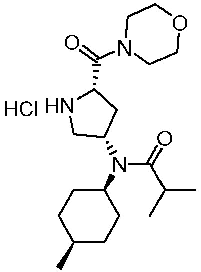

Tert-butyl (2*S*,4*S*)-4-(*N*((1*s*,4*R*)-4-methylcyclohexyl)isobutyramido)-2-(morpholine-4-carbonyl)pyrrolidine-1-carboxylate (1.87 g, 4.00 mmol) obtained in Step F above was dissolved in 30 mL of dichloromethane and cooled to 0°C, and 4 M hydrochloric acid 1,4-dioxane (2.15 mL, 8.59 mmol) was added thereto. After the mixture solution was stirred at room temperature for 16 hours, the reaction solution was concentrated under reduced pressure. The resulting solid was washed with ethyl ether and dried to obtain the title compound (1.22 g, 76%).
MS [M+H] = 366.4 (M+1)
¹H NMR (400 MHz, DMSO-d₆) δ 9.88 (brs, 1H), 8.12 (m, 1H), 4.51 (m, 1H), 4.20 (m, 1H), 3.63-3.35 (m, 10H), 3.30 (m, 1H), 2.85 (m, 1H), 2.51 (m, 1H), 2.01 (m, 1H), 1.91 (m, 1H), 1.76-1.60 (m, 4H), 1.53 (m, 2H), 1.40 (m, 2H), 0.99 (m, 9H)

### Preparation Example 3: Preparation of methyl (2S,4S)-4-(N-(4,4-dimethylcyclohexyl)pivalamido)pyrrolidine-2-carboxylate hydrochloride

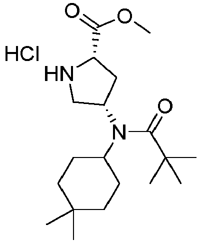

The title compound was obtained through the following Steps A and B.

### Step A: Preparation of 1-(tert-butyl) 2-methyl (2S,4S)-4-(N-(4,4-dimethylcyclohexyl)pivalamido)pyrrolidine-1,2-dicarboxylate

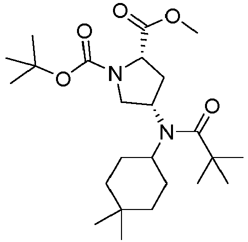

The title compound was obtained by the method disclosed in International Publication No. WO 2008/007930.
MS [M+Na] = 461.4 (M+23)
¹H NMR (400 MHz, CD₃OD) δ 4.34 (t, 1H), 3.90-3.70 (m, 2H), 3.73 (m, 3H), 3.45 (m, 2H), 2.74-2.61 (m, 1H), 2.30 (m, 1H), 1.83 (m, 2H), 1.53 (m, 4H), 1.45-1.40 (m, 9H), 1.40-1.30 (m, 2H), 1.24 (s, 9H), 0.99 (s, 3H), 0.94 (s, 3H)

### Step B: Preparation of methyl (2S,4S)-4-(N-(4,4-dimethylcyclohexyl)pivalamido)pyrrolidine-2-carboxylate hydrochloride

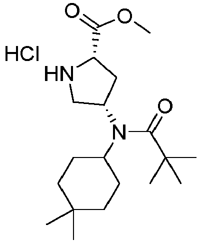

1-(*Tert*-butyl) 2-methyl (2*S*,4*S*)-4-(*N*-(4,4-dimethylcyclohexyl)pivalamido)pyrrolidine-1,2-dicarboxylate (1.67 g, 3.81 mmol) obtained in Step A was dissolved in 4 mL of ethyl acetate, and 4 M hydrochloric acid ethyl acetate solution (3.81 mL, 15.2 mmol) was added thereto and stirred at room temperature for 16 hours. The reaction solution was concentrated under reduced pressure to obtain the title compound (1.43 g, 99%).
MS [M+H] = 339.4 (M+1)
¹H NMR (400 MHz, CD₃OD) δ 4.41 (t, 1H), 4.24 (m, 1H), 3.86 (m, 1H), 3.83 (s, 3H), 3.42 (m, 2H), 2.66 (m, 1H), 2.22 (m, 1H), 1.77 (m, 2H), 1.51 (m, 4H), 1.34 (m, 2H), 1.23 (s, 9H), 0.99 (s, 3H), 0.93 (s, 3H)

### Preparation Example 4: Preparation of methyl (2S,4S)-4-(N-(4,4-dimethylcyclohexyl)isobutyramido)pyrrolidine-2-carboxylate hydrochloride

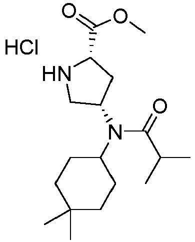

The title compound was obtained through the following Steps A and B.

### Step A: Preparation of 1-(tert-butyl) 2-methyl (2S,4S)-4-(N-(4,4-dimethylcyclohexyl)isobutyramido)pyrrolidine-1,2-dicarboxylate

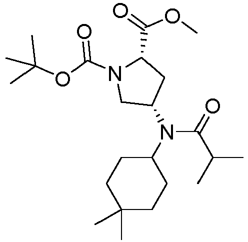

The title compound was obtained by the method disclosed in International Publication No. WO 2008/007930.
MS [M+Na] = 447.4 (M+23)
¹H NMR (400 MHz, CD₃OD) δ 4.31 (t, 1H), 3.93 (m, 1H), 3.83 (m, 1H), 3.75-3.68 (m, 3H), 3.62 (m, 1H), 3.45 (m, 1H), 2.81 (m, 1H), 2.26 (m, 1H), 1.80 (m, 2H), 1.50-1.35 (m, 14H), 1.21 (m, 2H), 1.04 (m, 6H), 0.96 (s, 3H), 0.92 (s, 3H)

### Step B: Preparation of methyl (2S,4S)-4-(N-(4,4-dimethylcyclohexyl)isobuiyramido)pyrrolidine-2-carboxylatehydrochloride

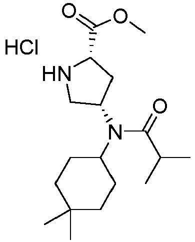

1-(*Tert*-butyl) 2-methyl (2*S*,4*S*)*-*4*-*(*N-*(4,4-dimethylcyclohexyl)isobutyramido)pyrrolidine-1,2-dicarboxylate (1.1 g, 2.6 mmol) obtained in Step A was dissolved in 2.5 mL of ethyl acetate, and 4 M hydrochloric acid ethyl acetate solution (2.5 mL, 10 mmol) was added thereto. The reaction solution was stirred at room temperature for 16 hours and concentrated under reduced pressure to obtain the title compound (0.93 g, 99%).
MS [M+H] = 325.4 (M+1)
¹H NMR (400 MHz, CD₃OD) δ 4.41 (t, 1H), 4.30 (m, 1H), 3.84 (s, 3H), 3.66 (m, 1H), 3.47 (m, 3H), 2.85 (m, 1H), 2.72 (m, 1H), 2.24 (m, 1H), 1.75 (m, 2H), 1.51 (m, 4H), 1.38 (m, 2H), 1.06 (m, 6H), 0.98 (s, 3H), 0.93 (s, 3H)

### Preparation Example 5: Preparation of (3S,4R)-1-(tert-butyl)-4-(4-chlorophenyl)pyrrolidine-3-carboxylic acid

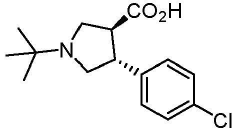

The title compound was obtained by the method disclosed in International Publication No. WO 2004/092126.
MS [M+H] = 282 (M+1)
¹H NMR (400 MHz, CD₃OD) δ 7.43-7.33 (m, 4H), 3.90-3.69 (m, 3H), 3.59 (m, 1H), 3.29 (m, 1H), 3.18-3.09 (m, 1H), 1.44 (s, 9H)

### Preparation Example 6: Preparation of (3S,4R)-1-(tert-butyl)-4-(2,4-difluorophenyl)pyrrolidine-3-carboxylic acid

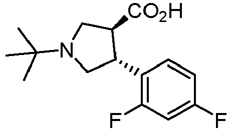

The title compound was obtained by the method disclosed in International Publication No. WO 2004/092126.
MS [M+H] = 284.2 (M+1)
¹H NMR (400 MHz, CD₃OD) δ 7.50 (m, 1H), 6.97 (m, 2H), 3.93-3.75 (m, 3H), 3.60 (m, 1H), 3.26 (m, 2H), 1.43 (s, 9H)

### Preparation Example 7: Preparation of (3S,4R)-1-(tert-butyl)-4-(4-chloro-2-fluorophenyl)pyrrolidine-3-carboxylic acid

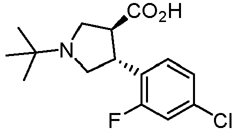

The title compound was obtained by the method disclosed in International Publication No. WO 2004/092126.
MS [M+H] = 300.3 (M+1)
¹H NMR (400 MHz, CD₃OD) δ 7.47 (t, 1H), 7.22 (m, 2H), 3.93-3.75 (m, 3H), 3.60 (m, 1H), 3.26 (m, 2H), 1.43 (s, 9H)

### Example 1: Preparation of N-((3S,5S)-1-((3S,4R)-1-(tert-butyl)-4-(2,4-difluorophenyl)pyrrolidine-3-carbonyl)-5-(morpholine-4-carbonyl)pyrrolidin-3-yl)-N-((1s,4R)-4-methylcyclohexyl)pivalamide hydrochloride

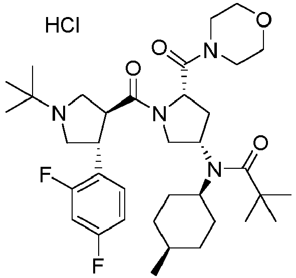

The title compound was obtained through the following Steps A and B.

### Step A: Preparation of N-((3S,5S)-1-((3S,4R)-1-(tert-bulyl)-4-(2,4-difluorophenyl)pyrrolidine-3-carbonyl)-5-(morpholine-4-carbonyl)pyrrolidin-3-yl)-N-((1s,4R)-4-methylcyclohexyl)pivalamide

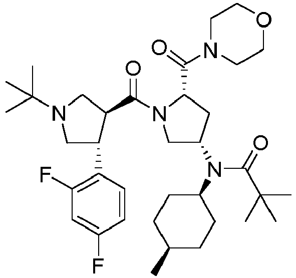

To *N*-((1*s*,4*R*)-4-methylcyclohexyl)-*N-*((3*S*,5*S*)-5-(morpholine-4-carbonyl)pyrrolidin-3-yl)pivalamide hydrochloride (0.50 g, 1.2 mmol) obtained in Preparation Example 1 and (3*S*,4*R*)-1-(*tert*-butyl)-4-(2,4-difluorophenyl)pyrrolidine-3-carboxylic acid (0.34 g, 1.2 mmol) obtained in Preparation Example 6, dimethylformamide (12 mL), 1-(3-dimethylaminopropyl)-3-ethylcarbodiimide hydrochloride (0.28 g, 1.5 mmol), 1-hydroxybenzotriazole hydrate (0.22 g, 1.5 mmol) and *N*,*N*-diisopropylethylamine (0.64 mL, 3.6 mmol) were added and stirred at room temperature for 16 hours. After the reaction solution was concentrated under reduced pressure, ethyl acetate was added. The reaction mixture was washed with an aqueous ammonium chloride solution and 1 M aqueous sodium hydroxide solution, and then dried over anhydrous sodium sulfate. The solid was filtered, and the filtrate was concentrated under reduced pressure and purified by chromatography to obtain the title compound (0.32 g, 40%).
MS [M+H] = 645.6 (M+1)
¹H NMR (400 MHz, CD₃OD) δ 7.58 (m, 1H), 6.95 (m, 2H), 4.76 (m, 1H), 4.18 (m, 1H), 3.84-3.40 (m, 15H), 3.11 (m, 1H), 2.89 (m, 1H), 2.12 (m, 1H), 1.95 (m, 1H), 1.79 (m, 1H), 1.65 (m, 4H), 1.44 (m, 2H), 1.28 (m, 2H), 1.19 (s, 9H), 1.17 (s, 9H), 1.03 (m, 3H)

### Step B: Preparation of N-((3S,5S)-1-((3S,4R)-1-(tert-butyl)-4-(2,4-difluorophenyl)pyrrolidine-3-carbonyl)-5-(morpholine-4-carbonyl)pyrrolidin-3-yl)-N-((1s,4R)-4-methylcyclohexyl)pivalamide hydrochloride

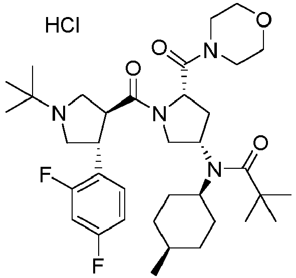

To *N*-((3*S*,5*S*)-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N*-((1*s*,4*R*)-4-methylcyclohexyl)pivalamide (0.32 g, 0.50 mmol) obtained in step A, 5 mL of dichloromethane and 4 M hydrochloric acid ethyl acetate solution (0.25 mL, 1.0 mmol) were added and stirred at room temperature for 20 minutes. The reaction solution was concentrated under reduced pressure, and diethyl ether was added thereto. The resulting solid was filtered and dried to obtain the title compound (0.23 g, 68%).
MS [M+H] = 645.6 (M+1)
¹H NMR (400 MHz, CD₃OD) δ 7.63 (m, 1H), 7.07 (m, 2H), 4.82 (m, 1H), 4.19 (m, 1H), 4.00-3.50 (m, 15H), 3.03 (m, 1H), 2.69 (m, 1H), 2.17 (m, 1H), 1.95 (m, 1H), 1.79 (m, 1H), 1.64 (m, 4H), 1.47 (s, 9H), 1.45-1.25 (m, 4H), 1.19 (s, 9H), 1.00 (m, 3H)

### Example 2: Preparation of N-((3S,5S)-1-((3S,4R)-1-(tert-butyl)-4-(2,4-difluorophenyl)pyrrolidine-3-carbonyl)-5-(morpholine-4-carbonyl)pyrrolidin-3-yl)-N-((1s,4R)-4-methylcyclohexyl)isobutyramide hydrochloride

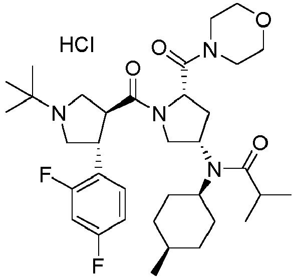

The title compound was obtained through the following Steps A and B.

### Step A: Preparation of N-((3S,5S)-1-((3S,4R)-1-(tert-bulyl)-4-(2,4-difluorophenyl)pyrrolidine-3-carbonyl)-5-(morpholine-4-carbonyl)pyrrolidin-3-yl)-N-((1s,4R)-4-methylcyclohexyl)isobutyramide

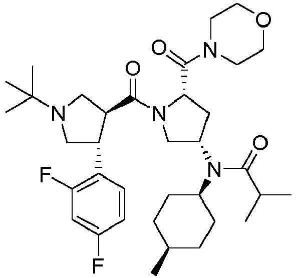

(3*S*,4*R*)-1-(*tert*-butyl)-4-(2,4-difluorophenyl)pyrrolidine-3-carboxylic acid (0.50 g, 1.76 mmol) obtained in Preparation Example 6 and *N-*((1*s*,4*R*)-4-methylcyclohexyl)-*N-*((3*S*,5*S*)-5-(morpholine-4-carbonyl)pyrrolidin-3-yl)isobutyramide hydrochloride (0.71 g, 1.76 mmol) obtained in Preparation Example 2 were used in the same manner as in Step A of Example 1 to obtain the title compound (0.10 g, 9%).
MS [M+H] = 631.5 (M+1)
¹H NMR (400 MHz, CD₃OD) δ 7.56 (m, 1H), 7.00 (m, 2H), 4.79 (m, 1H), 4.56 (m, 1H), 4.17 (m, 1H), 3.84 (m, 1H), 3.70-3.50 (m, 13H), 3.09 (m, 1H), 2.80 (m, 1H), 2.70 (m, 1H), 2.12 (m, 1H), 1.93 (m, 1H), 1.75-1.50 (m, 5H), 1.40 (m, 2H), 1.29 (s, 9H), 1.24 (m, 2H), 1.00 (m, 9H)

### Step B: Preparation of N-((3S,5S)-1-((3S,4R)-1-(tert-butyl)-4-(2,4-difluorophenyl)pyrrolidine-3-carbonyl)-5-(morpholine-4-carbonyl)pyrrolidin-3-yl)-N-((1s,4R)-4-methylcyclohexyl)isobutyramide hydrochloride

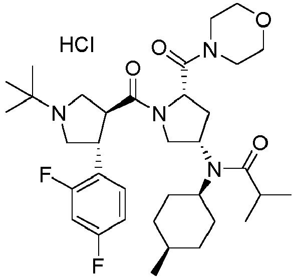

*N*-((3*S*,5S)-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N*-((1*s*,4*R*)-4-methylcyclohexyl)isobutyramide (0.10 g, 0.16 mmol) obtained in Step A was used in the same manner as in Step B of Example 1 to obtain the title compound (0.083 g, 78%).
MS [M+H] = 631.5 (M+1)
¹H NMR (400 MHz, CD₃OD) δ 7.63 (m, 1H), 7.07 (m, 2H), 4.80 (t, 1H), 4.21 (m, 1H), 3.94 (m, 2H), 3.85-3.50 (m, 13H), 3.03 (m, 1H), 2.82 (m, 1H), 2.70 (m, 1H), 2.15 (m, 1H), 1.95 (m, 1H), 1.80-1.59 (m, 5H), 1.47 (s, 9H), 1.40-1.20 (m, 4H), 1.01 (m, 9H)

### Example 3: Preparation of N-((3S,5S-1-((3S,4R)-1-(tert-butyl)-4-(4-chloro-2-fluorophenyl)pyrrolidine-3-carbonyl)-5-(morpholine-4-carbonyl)pyrrolidin-3-yl)-N-((1s,4R)-4-methylcyclohexyl)isobutyramide hydrochloride

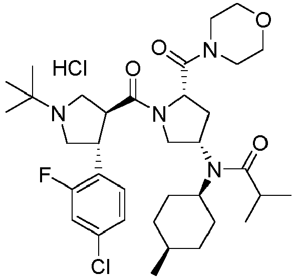

The title compound was obtained through the following Steps A and B.

### Step A: Preparation of N-((3S,5S)-1-((3S,4R)-1-(tert-bulyl)-4-(4-chloro-2-fluorophenyl)pyrrolidine-3-carbonyl)-5-(mopholine-4-carbonyl)pyrrolidin-3-yl)-N-((1s,4R)-4-methylcyclohexyl)isobutyramide

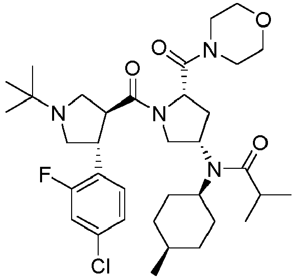

(3*S*,4*R*)-1-(*tert*-butyl)-4-(4-chloro-2-fluorophenyl)pyrrolidine-3-carboxylic acid (0.50 g, 1.67 mmol) obtained in Preparation Example 7 and *N-*((1*s*,4*R*)-4-methylcyclohexyl)-*N-*((3*S*,5*S*-5-(morpholine-4-carbonyl)pyrrolidin-3-yl)isobutyramide hydrochloride (0.67 g, 1.67 mmol) obtained in Preparation Example 2 were used in the same manner as in Step A of Example 1 to obtain the title compound (0.13 g, 12%). MS [M+H] = 647.5 (M+1)
¹H NMR (400 MHz, CD₃OD) δ 7.54 (t, 1H), 7.20 (m, 2H), 4.79 (m, 1H), 4.18 (m, 1H), 3.85 (m, 1H), 3.50-3.50 (m, 13H), 3.40 (m, 1H), 3.02 (m, 1H), 2.78 (m, 1H), 2.68 (m, 1H), 2.14 (m, 1H), 1.92 (m, 1H), 1.80-1.39 (m, 7H), 1.29-1.17 (m, 11H), 1.01 (m, 9H)

### Step B: Preparation of N-((3S,5S)-1-((3S,4R)-1-(tert-butyl)-4-(4-chloro-2-fluorophenyl)pyrrolidine-3-carbonyl)-5-(mopholine-4-carbonyl)pyrrolidin-3-yl)-N-((1s,4R)-4-methylcyclohexyl)isobutyramide hydrochloride

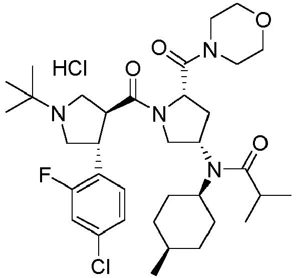

*N*-((3*S*,5*S*)-1-((3*S*,4*R*)-1-(*tert*-butyl)-4-(4-chloro-2-fluorophenyl)pyrrolidine-3-carbonyl)-5-(morpholine-4-carbonyl)pyrrolidin-3-yl)-*N*-((1*s*,4*R*)-4-methylcyclohexyl)isobutyramide (0.13 g, 0.20 mmol) obtained in Step A was used in the same manner as in Step B of Example 1 to obtain the title compound (0.1 g, 80%).
MS [M+H] = 647.5 (M+1)
¹H NMR (400 MHz, CD₃OD) δ 7.60 (m, 1H), 7.30 (m, 2H), 4.81 (m, 1H), 4.22 (m, 1H), 4.04-3.90 (m, 2H), 3.86-3.41 (m, 13H), 3.12 (m, 1H), 2.83 (m, 1H), 2.70 (m, 1H), 2.20 (m, 1H), 1.95 (m, 1H), 1.80-1.55 (m, 5H), 1.45-1.27 (m, 4H), 1.47 (s, 9H), 1.02 (m, 9H)

### Example 4: Preparation of N-((3S,5S-1-((3S,4R)-1-(tert-butyl)-4-(4-chloro-2-fluorophenyl)pyrrolidine-3-carbonyl)-5-(morpholine-4-carbonyl)pyrrolidin-3-yl)-N-((1s,4R)-4-methylcyclohexyl)pivalamide hydrochloride

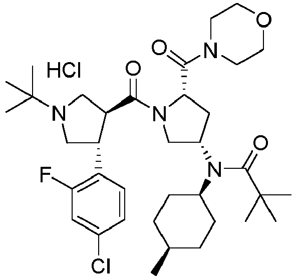

The title compound was obtained through the following Steps A and B.

### Step A: Preparation of N-((3S,5S)-1-((3S,4R)-1-(tert-bulyl)-4-(4-chloro-2-fluorophenyl)pyrrolidine-3-carbonyl)-5-(morpholine-4-carbonyl)pyrrolidin-3-yl)-N-((1s,4R)-4-methylcyclohexyl)pivalamide

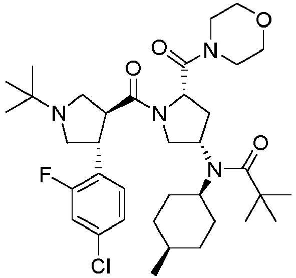

(3*S*,4*R*)-1-(*tert*-butyl)-4-(4-chloro-2-fluorophenyl)pyrrolidine-3-carboxylic acid (0.36 g, 1.21 mmol) obtained in Preparation Example 7 and *N-*((1*s*,4*R*)-4-methylcyclohexyl)-*N-*((3*S*,5*S*)-5-(morpholine-4-carbonyl)pyrrolidin-3-yl)pivalamide hydrochloride (0.50 g, 1.21 mmol) obtained in Preparation Example 1 were used in the same manner as in Step A of Example 1 to obtain the title compound (0.45 g, 56%).
MS [M+H] = 661.6 (M+1)
¹H NMR (400 MHz, CD₃OD) δ 7.55 (m, 1H), 7.18 (m, 2H), 4.79 (m, 1H), 4.18 (m, 1H), 3.90-3.20 (m, 15H), 3.08 (m, 1H), 2.70 (m, 1H), 2.12 (m, 1H), 1.93 (m, 1H), 1.81-1.60 (m, 5H), 1.47 (m, 2H), 1.30-1.10 (m, 2H), 1.20 (s, 9H), 1.15 (s, 9H), 1.03 (m, 3H)

### Step B: Preparation of N-((3S,5S)-1-((3S,4R)-1-(tert-butyl)-4-(4-chloro-2-fluorophenyl)pyrrolidine-3-carbonyl)-5-(morpholine-4-carbonyl)pyrrolidin-3-yl)-N-((1s,4R)-4-methylcyclohexyl)pivalamide hydrochloride

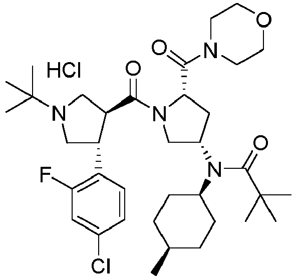

*N*-((3*S*,5*S*)-1-((3*S*,4*R*)-1-(*tert*-butyl)-4-(4-chloro-2-fluorophenyl)pyrrolidine-3-carbonyl)-5-(morpholine-4-carbonyl)pyrrolidin-3-yl)-*N*-((1*s*,4*R*)-4-methylcyclohexyl)pivalamide (0.25 g, 0.38 mmol) obtained in Step A was used in the same manner as in Step B of Example 1 to obtain the title compound (0.18 g, 68%).
MS [M+H] = 661.6 (M+1)
¹H NMR (400 MHz, CD₃OD) δ 7.60 (t, 1H), 7.30 (m, 2H), 4.80 (m, 1H), 4.20 (m, 1H), 4.04-3.90 (m, 2H), 3.86-3.40 (m, 13H), 3.12 (m, 1H), 2.67 (m, 1H), 2.16 (m, 1H), 1.94 (m, 1H), 1.82-1.60 (m, 5H), 1.47 (s, 9H), 1.45-1.20 (m, 4H), 1.20 (s, 9H), 1.03 (m, 3H)

### Example 5: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-(4,4-dimethylcyclohexyl)isobutyramide hydrochloride

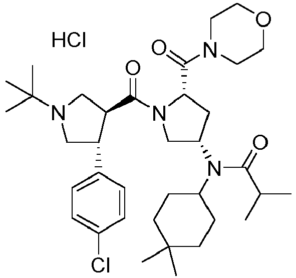

The title compound was obtained through the following Steps A, B, C and D.

### Step A: Preparation of methyl (2S,4S)-1-((3S,4R)-1-(tert-butyl)-4-(4-chlorophenyl)pyrrolidine-3-carbonyl)-4-(N-(4,4-dimethylcyclohexyl)isobutyramido)pyrrolidine-2-carboxylate

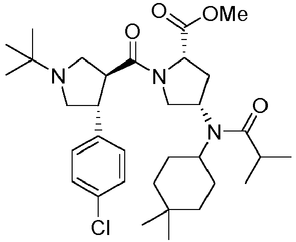

(3*S*,4*R*)-1-(*tert*-butyl)-4-(4-chlorophenyl)pyrrolidine-3-carboxylic acid (0.73 g, 2.58 mmol) obtained in Preparation Example 5 and methyl (2*S*,4*S*)-4-(*N*-(4,4-dimethylcyclohexyl)isobutyramido)pyrrolidine-2-carboxylate hydrochloride (0.93 g, 2.58 mmol) obtained in Preparation Example 4 were used in the same manner as in Step A of Example 1 to obtain the title compound (0.49 g, 32%).
MS [M+H] = 588.5 (M+1)
¹H NMR (400 MHz, CD₃OD) δ 7.30 (m, 4H), 4.42 (t, 1H), 4.01 (t, 1H), 3.69 (s, 3H), 3.65-3.50 (m, 3H), 3.34-3.20 (m, 2H), 3.13-3.04 (m, 2H), 2.87 (m, 1H), 2.79 (m, 1H), 2.67 (m, 1H), 2.15 (m, 1H), 1.69 (m, 1H), 1.56 (m, 1H), 1.50-1.26 (m, 7H), 1.16 (s, 9H), 1.00 (m, 6H), 0.93 (m, 6H)

### Step B: Preparation of (2S,4S)-1-((3S,4R)-1-(tert-butyl)-4-(4-chlorophenyl)pyrrolidine-3-carbonyl)-4-(N-(4,4-dimethylcyclohexyl)isobutyramido)pyrrolidine-2-carboxylic acid

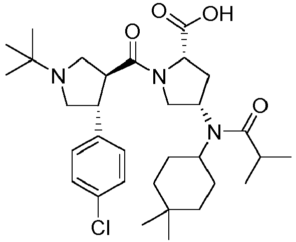

Methyl (2*S*,4*S*)-1-((3*S*,4*R*)-1-(*tert*-butyl)-4-(4-chlorophenyl)pyrrolidine-3-carbonyl)-4-(*N*-(4,4-dimethylcyclohexyl)isobutyramido)pyrrolidine-2-carboxylate (0.49 g, 0.83 mmol) obtained in Step A was dissolved in methanol (2.8 mL) and cooled to 0°C, and 6 M aqueous sodium hydroxide solution (0.7 mL, 4.2 mmol) was added thereto. The mixture solution was stirred at room temperature for 16 hours and then concentrated under reduced pressure. Water was added thereto, the pH was adjusted to 4, and the reaction mixture was concentrated under reduced pressure. The mixture was dissolved in dichloromethane, and the solid was removed by filtration. The filtrate was concentrated under reduced pressure to obtain the title compound (0.47 g, 98%).
MS [M+H] = 574.4 (M+1)
¹H NMR (400 MHz, CD₃OD) δ 7.40 (m, 4H), 4.41 (m, 1H), 4.13-3.65 (m, 5H), 3.60-3.35 (m, 3H), 2.96 (m, 1H), 2.82-2.69 (m, 2H), 2.18 (m, 1H), 1.73-1.55 (m, 2H), 1.45 (s, 9H), 1.42-1.20 (m, 7H), 1.00 (m, 6H), 0.94 (m, 6H)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-(4,4-dimethylcyclohexyl)isobutyramide

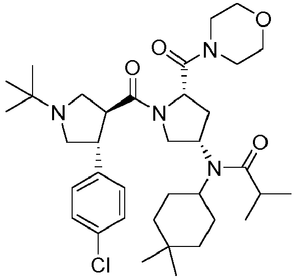

(2*S*,4*S*)-1-((3*S*,4*R*)-1-(*tert*-butyl)-4-(4-chlorophenyl)pyrrolidine-3-carbonyl)-4-(*N*-(4,4-dimethylcyclohexyl)isobutyramido)pyrrolidine-2-carboxylic acid (0.47 g, 0.82 mmol) obtained in Step A and morpholine (0.071 mL, 0.82 mmol) were used in the same manner as in Step A of Example 1 to obtain the title compound (0.41 g, 78%).
MS [M+H] = 643.4 (M+1)
¹H NMR (400 MHz, CD₃OD) δ 7.34 (m, 2H), 7.30 (m, 2H), 4.77 (m, 1H), 4.12 (m, 1H), 3.80-3.30 (m, 15H), 3.05 (m, 1H), 2.77 (m, 1H), 2.64 (m, 1H), 2.09 (m, 1H), 1.80-1.58 (m, 2H), 1.50-1.24 (m, 7H), 1.14 (s, 9H), 0.99 (m, 6H), 0.95 (m, 6H)

### Step D: Preparation of N-((3S,5S)-1-((3S,4R)-1-(tert-butyl)-4-(4-chiorophenyl)pyrrolidine-3-carbonyl)-5-(morpholine-4-carbonyl)pyrrolidin-3-yl)-N-(4,4-dimethylcyclohexyl)isobutyramide hydrochloride

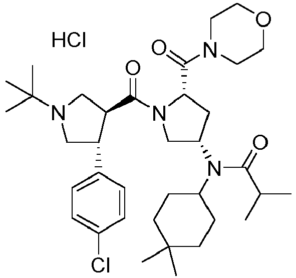

*N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-(4,4-dimethylcyclohexyl)isobutyramide (0.42 g, 0.65 mmol) obtained in Step C was used in the same manner as in Step B of Example 1 to obtain the title compound (0.37 g, 83%).
MS [M+H] = 643.4 (M+1)
¹H NMR (400 MHz, CD₃OD) δ 7.44 (m, 4H), 4.81 (m, 1H), 4.22 (m, 1H), 3.90 (m, 1H), 3.80-3.40 (m, 14H), 2.99 (m, 1H), 2.82 (m, 1H), 2.68 (m, 1H), 2.16 (m, 1H), 1.80-1.60 (m, 2H), 1.40-1.20 (m, 7H), 1.47 (s, 9H), 1.05 (m, 6H), 0.96 (m, 6H)

### Example 6: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-(4,4-dimethylcyclohexyl)pivalamide hydrochloride

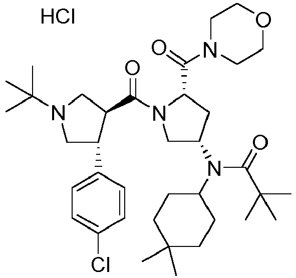

The title compound was obtained through the following Steps A, B, C and D.

### Step A: Preparation of methyl (2S,4S)-1-((3S,4R)-1-(tert-butyl)-4-(4-chlorophenyl)pyrrolidine-3-carbonyl)-4-(N-(4,4-dimethylcyclohexyl)pivalamido)pyrrolidine-2-carboxylate

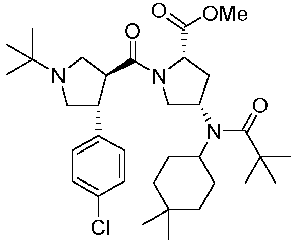

(3*S*,4*R*)-1-(*tert*-butyl)-4-(4-chlorophenyl)pyrrolidine-3-carboxylic acid (0.60 g, 2.13 mmol) obtained in Preparation Example 5 and methyl (2*S*,4*S*)-4-(*N*-(4,4-dimethylcyclohexyl)pivalamido)pyrrolidine-2-carboxylate hydrochloride (0.80 g, 2.13 mmol) obtained in Preparation Example 3 were used in the same manner as in Step A of Example 1 to obtain the title compound (0.63 g, 49%).
MS [M+H] = 602.5 (M+1)
¹H NMR (400 MHz, CD₃OD) δ 7.34 (m, 4H), 4.47 (t, 1H), 3.98 (m, 1H), 3.80 (m, 1H), 3.71 (s, 3H), 3.60 (m, 2H), 3.48-3.34 (m, 3H), 3.17 (m, 1H), 3.10 (m, 1H), 2.63 (m, 1H), 2.16 (m, 1H), 1.73 (m, 1H), 1.58 (m, 1H), 1.52-1.38 (m, 3H), 1.36-1.20 (m, 4H), 1.23 (s, 9H), 1.17 (s, 9H), 0.97 (s, 3H), 0.94 (s, 3H)

### Step B: Preparation of (2S,4S)-1-((3S,4R)-1-(tert-butyl)-4-(4-chlorophenyl)pyrrolidine-3-carbonyl)-4-(N-(4,4-dimethylcyclohexyl)pivalamido)pyrrolidine-2-carboxylic acid

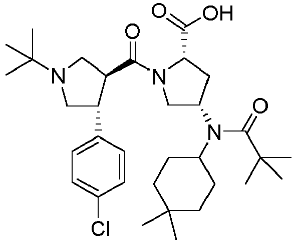

Methyl (2*S*,4*S*)-1-((3*S*,4*R*)-1-(*tert*-butyl)-4-(4-chlorophenyl)pyrrolidine-3-carbonyl)-4-(A-(4,4-dimethylcyclohexyl)pivalamido)pyrrolidine-2-carboxylate (0.63 g, 1.05 mmol) obtained in Step A was used in the same manner as in Step B of Example 5 to obtain the title compound (0.51 g, 83%).
MS [M+H] = 588.5 (M+1)
¹H NMR (400 MHz, CD₃OD) δ 7.42 (m, 4H), 4.41 (m, 1H), 4.06 (m, 1H), 3.90 (m, 2H), 3.80-3.60 (m, 3H), 3.47 (m, 2H), 2.94 (m, 1H), 2.72 (m, 1H), 2.18 (m, 1H), 1.71 (m, 1H), 1.61 (m, 1H), 1.50-1.20 (m, 7H), 1.47 (s, 9H), 1.19 (s, 9H), 0.98 (s, 3H), 0.94 (s, 3H)

### Step C: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-(4,4-dimethylcyclohexyl)pivalamide

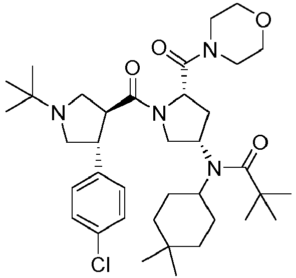

(2*S*,4*S*)-1-((3*S*,4*R*)-1-(*tert*-butyl)-4-(4-chlorophenyl)pyrrolidine-3-carbonyl)-4-(*N*-(4,4-dimethylcyclohexyl)pivalamido)pyrrolidine-2-carboxylic acid (0.51 g, 0.87 mmol) obtained in Step B and morpholine (0.076 mL, 0.87 mmol) were used in the same manner as in Step A of Example 1 to obtain the title compound (0.44 g, 77%).
MS [M+H] = 657.6 (M+1)
¹H NMR (400 MHz, CD₃OD) δ 7.36 (m, 2H), 7.30 (m, 2H), 4.77 (m, 1H), 4.14 (m, 1H), 3.80-3.20 (m, 15H), 3.05 (m, 1H), 2.68 (m, 1H), 2.09 (m, 1H), 1.75 (m, 1H), 1.61 (m, 1H), 1.51-1.24 (m, 7H), 1.15 (m, 18H), 0.97 (m, 6H)

### Step D: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-(4,4-dimethylcyclohexyl)pivalamide hydrochloride

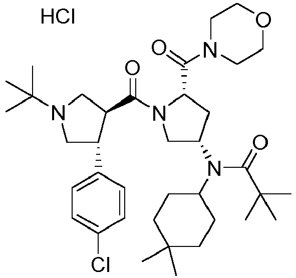

*N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-(4,4-dimethylcyclohexyl)pivalamide (0.44 g, 0.67 mmol) obtained in Step C was used in the same manner as in Step B of Example 1 to obtain the title compound (0.39 g, 84%).
MS [M+H] = 657.6 (M+1)
¹H NMR (400 MHz, CD₃OD) δ 7.45 (m, 4H), 4.81 (m, 1H), 4.16 (m, 1H), 4.06-3.85 (m, 2H), 3.84-3.40 (m, 13H), 2.98 (m, 1H), 2.67 (m, 1H), 2.13 (m, 1H), 1.75 (m, 1H), 1.63 (m, 1H), 1.47 (s, 9H), 1.50-1.20 (m, 7H), 1.18 (s, 9H), 0.96 (m, 6H)

### Comparative Example 1: Preparation of N-((3S,5S)-1-((3S,4R)-1-(tert-butyl)-4-(2,4-difluorophenyl)pyrrolidine-3-carbonyl)-5-(morpholine-4-carbonyl)pyrrolidin-3-yl)-N-(4,4-dimethylcyclohexyl)acetamide hydrochloride (A95)

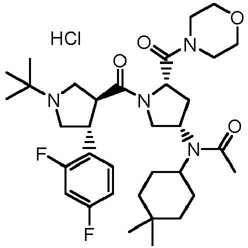

The A95 compound of International Publication No. WO 2008/007930 was obtained by the same method as disclosed therein.

### Comparative Example 2: Preparation of N-((3S,5S)-1-((3S,4R)-1-(tert-butyl)-4-(4-chlorophenyl)pyrrolidine-3-carbonyl)-5-(morpholine-4-carbonyl)pyrrolidin-3-yl)-N-(4,4-dimethylcyclohexyl)acetamide hydrochloride (A96)

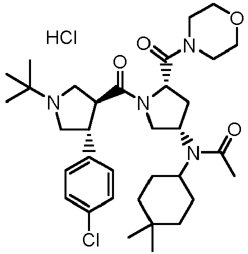

The A96 compound of International Publication No. WO 2008/007930 was obtained by the same method as disclosed therein.

### Experimental Example 1: Luciferase Assay

In order to measure the agonist ability against MC4R (melanocortin-4 receptor), a cell line that permanently expresses the luciferase gene (CRE-LUC) under the control of MC4R and CRE (cAMP response element) was established. After a mammalian cell expression vector (pCDNA3 (Neo)) (Invitrogen) containing the MC4R gene was prepared, human embryonic kidney (HEK) cell lines were transformed by using Lipofectamine 2000 (Invitrogen) together with a vector (pCRE-Luc)(Stratagen) expressing a luciferase gene (CRE-LUC) under the control of a cAMP response element (CRE). Transformed cell lines (HEK MC4R-Luc) were incubated in a 37°C incubator in the presence of 5% CO₂ for 24 hours by using Dulbecco's Modified Eagles Medium (DMEM) containing 10% Heat-Inactivated Fetal Bovine Serum (GIBCO/BRL). The cell lines were incubated for four days in the presence of Dulbecco's Modified Eagles Medium (DMEM) containing 10 ml of selection medium (10% Heat-Inactivated Fetal Bovine Serum (GIBCO/BRL), 100 unit/ml of penicillin (GIBCO/BRL), 100 unit/ml of streptomycin (GIBCO/BRL), 800 µg/ml of Geneticin (G418) (GIBCO/BRL). The process of removing cells killed by the selection medium by replacing the medium with 10 ml of a new selection medium was repeated three times, once every 4 days. Individual colonies formed by the finally selected and propagated clones were transferred under a microscope to a 24-well cell culture plate containing 1 ml of selection medium per well and incubated for 4 days. Forskolin (SIGMA) was treated to a final concentration of 10 µM, and then incubated for five hours in a 37°C incubator in the presence of 5% CO₂. Each well was treated with 50 µl of a Bright-Glo luciferase reagent (Promega) and left at room temperature for 15 minutes, and then the luminescence of each well was measured by using a luminometer (Victor). Clones exhibiting luminescence of 100 times or more of the basic value by treatment with Forskolin were selected and used to measure the MC4R agonist ability of each compound.

HEK MC4R-Luc cells were added to each well of a 96-well luminometer cell culture plate (Costar) to a size of 2.5 × 10⁴ cells in 100 µl of a culture medium and then incubated in a 37°C incubator in the presence of 6% CO₂ for 18 hours. The MCR agonist diluted at each step concentration by using the above culture medium was treated so that the final DMSO concentration did not exceed 1%, and then incubated for five hours in a 37°C incubator in the presence of 6% CO₂. Each well was treated with 50 µl of a Bright-Glo luciferase reagent (Promega) and left at room temperature for five minutes, and then the luminescence of each well was measured by using a luminometer (Victor). The amount of luminescence induced by the agonist diluted at each step concentration was converted into a relative % value with respect to the amount exhibited by a 10 µM NDP-α-MSH treatment. EC_{0.5} MSH is expressed as a concentration that induces 50% of the maximum amount of luminescence that can be induced by NDP-α-MSH, and EC₅₀ is expressed as a concentration that induces 50% of the maximum amount of luminescence that can be induced by each agonist. The measurements were measured using statistical software (Prizm).

Table 1 shows the results of measuring the agonist ability of MC4R of each compound obtained by the above experiments in EC₅₀ (nM) units.

**[Table 1]**

| **Luciferase assay, EC₅₀ (nM)** | |
|---|---|
| **Compound** | **MC4R** |
| Example 1 | 0.629 |
| Example 2 | 2.472 |
| Example 3 | 0.922 |
| Example 4 | 0.555 |
| Example 5 | 3.824 |
| Example 6 | 4.038 |
| Comparative Example 1 (A95) | 14.69 |
| Comparative Example 2 (A96) | 5.607 |

As shown in Table 1, it was confirmed that, among the well-known melanocortin receptors *in vivo,* with respect to the melanocortin-4 receptor (MC4R) involved in energy metabolism and weight control *in vivo,* the compounds of the examples have more excellent MC4R agonist ability than the compounds of the comparative examples (A95 and A96).

### Experimental Example 2: cAMP Assay

The melanocortin receptor is a type of G-protein coupled receptor (GPCR), and the main role of G-protein is to activate secondary transducers to regulate cellular responses to many physiological stimuli through signal transduction. MC4R is a Gs-coupled receptor, and it is known that if MC4R interacts with an agonist, adenylate cyclase (AC) is activated to increase the concentration of cyclic AMP (cAMP), which is one of the secondary transducers in cells. Therefore, it is possible to evaluate the activity of melanocortin receptors by measuring the generation of cAMP signals.

After cAMP Hunter Gs-coupled cell lines (CHO-K1 cell line) in which each of MC1R, MC3R, MC4R and MC5R was overexpressed were established so that the increase in the cAMP level in cells due to agonist reaction was measurable, the cells were inoculated into each well of a white cell culture plate and incubated for 24 hours in a 37°C incubator in the presence of 5% CO₂. After the incubation, the medium was removed, and 15 µl of a 2:1 HBBS/10 mM HEPES:cAMP XS+Ab reagent was added. After 5 µl of the sample diluted 4 times with buffer was added, the vehicle concentration was set to 1%, and the MC4R agonist compound diluted at each step concentration was added, followed by reaction at 37°C for 30 minutes. The activity (%) of each agonist compound is expressed as 100% × (average RLU value of sample - average RLU value of vehicle control) / (average RLU value of max control - average RLU value of vehicle control), and the value was analyzed by CBIS data analysis suite (ChemInnovation, CA).

Table 2 shows the results of measuring the agonist ability of melanocortin receptors of the compounds obtained by the above experiments in EC₅₀ (nM) units.

**[Table 2]**

| **cAMP assay, EC₅₀ (nM)** | |
|---|---|
| **Compound** | **MC4R** |
| Example 1 | 58.9 |
| Example 2 | 59.6 |
| Example 3 | 49.6 |
| Example 4 | 26.2 |
| Example 6 | 51.4 |
| Comparative Example 1 (A95) | 335.3 |
| Comparative Example 2 (A96) | 150.0 |

As shown in Table 2, it was confirmed that, among the well-known melanocortin receptors *in vivo,* with respect to the melanocortin-4 receptor (MC4R) involved in energy metabolism and weight control *in vivo,* the compounds of the examples have more excellent receptor agonist ability than the compounds of the comparative examples (A95 and A96).

### Experimental Example 3: β-arrestin Assay

The melanocortin receptor is a type of G-protein coupled receptor (GPCR) and regulates various physiological responses by transducing signals from many neurotransmitters. When GPCR is phosphorylated, β-arrestin is bound to the phosphorylated part of the receptor and plays an important role in activating various signaling pathways in cells through interaction with other proteins. It is known that, when the melanocortin receptor interacts with an agonist, β-arrestin is mobilized and is involved in the β-arrestin-mediated signaling pathway. Therefore, the activity of the melanocortin receptor can be evaluated through the measurement of β-arrestin.

A Pathhunter eXpress β-arrestin cell line (U2OS cell line) in which Prolink (PK)-tagged MC1R, MC3R, MC4R, MC5R and Enzyme acceptor (EA)-tagged β-arrestin were expressed together was established. When the MCR-PK portion of this cell line is activated, β-arrestin-EA is mobilized, and enzyme acceptor (EA) and Prolink (PK), which are the β-galactosidase enzyme fragments, interact. The activated enzyme hydrolyzes the substrate by β-galactosidase activity to produce a chemiluminescent signal, so that the activity can be measured. After the Pathhunter eXpress β-arrestin cell line (U2OS cell line) was incubated, the cells were inoculated into each well of the cell culture plate and incubated for 48 hours in a 37°C incubator in the presence of 5% CO₂. After the incubation, 5 µl of the sample diluted 5 times with buffer was added, the vehicle concentration was set to 1%, and the MC4R agonist compound diluted at each step concentration was added, followed by reaction at 37°C for 90 minutes. The activity (%) of each agonist compound is expressed as 100% × (average RLU value of sample - average RLU value of vehicle control) / (average maximum value of control ligand - average RLU value of vehicle control), and the value was analyzed by CBIS data analysis suite (ChemInnovation, CA).

Table 3 shows the results of measuring the activity ability of the melanocortin receptor of each compound obtained by the above experiments in EC₅₀ (nM) units.

**[Table 3]**

| **β-arrestin assay, EC₅₀ (nM)** | |
|---|---|
| **Compound** | **MC4R** |
| Example 1 | 8.02 |
| Example 2 | 15.0 |
| Example 3 | 4.5 |
| Example 4 | 4.2 |
| Example 6 | 13.0 |
| Comparative Example 1 (A95) | 176.4 |
| Comparative Example 2 (A96) | 44.3 |

As shown in Table 3, it was confirmed that, among the well-known melanocortin receptors *in vivo,* with respect to the melanocortin-4 receptor (MC4R) involved in energy metabolism and weight control *in vivo,* the compounds of the examples have more excellent receptor agonist ability than the compounds of the comparative examples (A95 and A96).

### Experimental Example 4: Binding Affinity

Five subtypes of melanocortin receptor (MCR) *in vivo* are known, and it is known that MC4R, which is Subtype 4, is involved in energy metabolism and weight control. Since other MCR subtypes are involved in the regulation of various functions *in vivo* such as skin pigmentation, energy homeostasis, and exocrine functions, securing selectivity for MC4R of MC4R agonist compounds is very important in preventing possible side effects in the future. Therefore, the receptor binding abilities of MC4R agonists for each MCR subtype were measured.

After the CHO-K1 cell line expressing human recombinant MC1R and the HEK-293 cell lines expressing MC3R, MC4R and MC5R were established, membranes were collected from each cell line. In a 96-well cell culture plate, 3 µg MC1R membrane and 0.04 nM ¹²⁵I-NDP-α-MSH per well were reacted at 37°C for two hours. 3 µg MC3R and MC5R membrane and 0.035 nM ¹²⁵I-NDP-α-MSH were reacted at 37°C for one hour, and 3.12 µg MC4R membrane and 0.02 nM ¹²⁵I-NDP-α-MSH were reacted at 37°C for two hours. At this time, 25 mM HEPES-KOH adsorption buffer (pH 7.0) containing MCR agonist diluted at each step concentration was added to each well and reacted. The reacted solution was transferred to a filter and washed with an adsorption buffer, and then radioactivity was measured. The value excluding the non-specific binding amount in the presence of 1 µM (MC1R) and 3 µM (MC3R, MC4R, MC5R) of NDP-α-MSH from each total binding amount was used as specific binding amounts of ¹²⁵I-NDP-α-MSH. The degree to which the ¹²⁵I-NDP-α-MSH specific binding was inhibited by the agonist diluted at each step concentration was measured. IC50 was expressed as the concentration of each agonist that inhibited specific binding of 50% of ¹²⁵I-NDP-α-MSH.

Tables 4-1 and 4-2 show the results of measuring the binding of melanocortin receptors of the compounds obtained by the above experiment in Ki (nM) units.

**[Table 4-1]**

| **Binding affinity, Ki (nM)** | |
|---|---|
| **Compound** | **MC4R** |
| Example 1 | 74 |
| Example 2 | 240 |
| Example 4 | 30 |
| Comparative Example 1 (A95) | 1450 |
| Comparative Example 2 (A96) | 410 |

**[Table 4-2]**

| **Binding affinity, Ki (nM)** | |
|---|---|
| **Compound** | **MC4R** |
| Example 3 | 86 |
| Example 6 | 32 |
| Comparative Example 1 (A95) | 1780 |
| Comparative Example 2 (A96) | 530 |

As shown in Tables 4-1 and 4-2, it was confirmed that, among the well-known melanocortin receptors *in vivo,* with respect to the melanocortin-4 receptor (MC4R) involved in energy metabolism and weight control *in vivo,* the compounds of the examples have more excellent receptor binding ability than the compounds of the comparative examples (A95 and A96).

## Claims

1. A compound of the following Formula 1, or a pharmaceutically acceptable salt or isomer thereof: 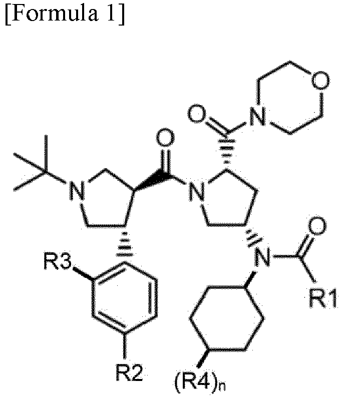 wherein
R1 is C₂-C₅ alkyl;
R2 is halo;
R3 is hydrogen or halo;
R4 is C₁-C₃ alkyl; and
n is an integer of 1 or 2;
provided that n is 2, when R2 is chloride and R3 is hydrogen.

2. The compound, or a pharmaceutically acceptable salt or isomer thereof according to Claim 1, wherein R1 is C₂-C₄ alkyl.

3. The compound, or a pharmaceutically acceptable salt or isomer thereof according to Claim 2, wherein the compound of Formula 1 is selected from the following group:
*N*-((3*S*,5*S*)-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N*-((1*s*,4*R*)-4-methylcyclohexyl)pivalamide;
*N*-((3*S*,5*S*)-1-((3*S*,4*R*)-1-(*tert*-butyl)-4-(2,4-difluorophenyl)pyrrolidine-3-carbonyl)-5-(morpholine-4-carbonyl)pyrrolidin-3-yl)-*N*-((1*s*,4*R*)-4-methylcyclohexyl)isobutyramide;
*N*-((3*S*,5*S*)-1-((3*S*,4*R*)-1-(*tert*-butyl)-4-(4-chloro-2-fluorophenyl)pyrrolidine-3-carbonyl)-5-(morpholine-4-carbonyl)pyrrolidin-3-yl)-*N*-((1*s*,4*R*)-4-methylcyclohexyl)isobutyramide;
*N*-((3*S*,5*S*)-1-((3*S*,4*R*)-1-(*tert*-butyl)-4-(4-chloro-2-fluorophenyl)pyrrolidine-3-carbonyl)-5-(morpholine-4-carbonyl)pyrrolidin-3-yl)-*N*-((1*s*,4*R*)-4-methylcyclohexyl)pivalamide;
*N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-(4,4-dimethylcyclohexyl)isobutyramide; and
*N*-((3*S*,5*S*)-1-((3*S*,4*R*)-1-(*tert*-butyl)-4-(4-chlorophenyl)pyrrolidine-3-carbonyl)-5-(morpholine-4-carbonyl)pyrrolidin-3-yl)-*N*-(4,4-dimethylcyclohexyl)pivalamide.

4. The compound, or a pharmaceutically acceptable salt or isomer thereof according to Claim 1, wherein the pharmaceutically acceptable salt is selected from the group consisting of hydrochloric acid, sulfuric acid, nitric acid, phosphoric acid, hydrobromic acid and hydroiodic acid.

5. The compound, or a pharmaceutically acceptable salt or isomer thereof according to Claim 4, wherein the pharmaceutically acceptable salt is hydrochloride.

6. A melanocortin-4 receptor agonistic pharmaceutical composition comprising the compound of Formula 1, or a pharmaceutically acceptable salt or isomer thereof as defined in any one of Claims 1 to 5, together with a pharmaceutically acceptable carrier.

7. The melanocortin receptor agonistic pharmaceutical composition according to Claim 6, which is for the prevention or treatment of obesity.

8. The melanocortin receptor agonistic pharmaceutical composition according to Claim 6, which is for the prevention or treatment of diabetes.

9. The melanocortin receptor agonistic pharmaceutical composition according to Claim 6, which is for the prevention or treatment of inflammation.

10. The melanocortin receptor agonistic pharmaceutical composition according to Claim 6, which is for the prevention or treatment of erectile dysfunction.
